(19) Europäisches Patentamt
European Patent Office
Office européen des brevets

(11) Numéro de publication: **0 236 251**
**A2**

(12) **DEMANDE DE BREVET EUROPEEN**

(21) Numéro de dépôt: **87440012.0**

(22) Date de dépôt: **04.03.87**

(51) Int. Cl.⁴: **C 07 D 217/04**, A 61 K 31/47

(30) Priorité: **05.03.86 FR 8603201**

(43) Date de publication de la demande: **09.09.87**
**Bulletin 87/37**

(84) Etats contractants désignés: **AT BE CH DE ES FR GB GR IT LI LU NL SE**

(71) Demandeur: **UNIVERSITE LOUIS PASTEUR (STRASBOURG I), 4, rue Blaise Pascal, F-67070 Strasbourg Cédex (FR)**

(72) Inventeur: **Kanmacher, Isabelle, 12, rue Hardouin Mansart Mittelhausbergen, F-67200 Strasbourg (FR)**
Inventeur: **Stambach, Jean-François, 10, rue d'Andlau, F-67000 Strasbourg (FR)**
Inventeur: **Jung, Louis, 205, route d'Oberhausbergen, F-67200 Strasbourg (FR)**
Inventeur: **Schott, Christa, 7, square du Château, F-67300 Schiltigheim (FR)**
Inventeur: **Stoclet, Jean-Claude, 13, boulevard Jean-Sébastien Bach, F-67000 Strasbourg (FR)**
Inventeur: **Heitz, Christiane, 3, rue des Bouvreuils, F-67100 Strasbourg (FR)**

(74) Mandataire: **Nuss, Pierre, 10, rue Jacques Kablé, F-67000 Strasbourg (FR)**

(54) **Dérivés des (nitro-2 ou amino-2 benzyl)-2 tétrahydro-1,2,3,4 isoquinoléines, ainsi que les procédés d'obtention et les compositions pharmaceutiques les contenant.**

(57) Des dérivés de la nitro-2 ou amino-2 benzyltétrahydro-1,2,3,4 isoquinoléine ont été préparés et sont caractérisés par la structure suivante:

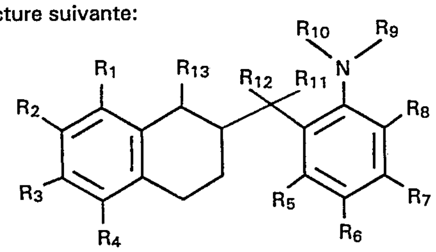

dans laquelle chacun des substituants R1, R2, R3, R4, R5, R6, R7 ou R8 peut représenter soit un atome d'hydrogène, soit un groupe alkyle linéaire ou ramifié, soit un groupe alcoxy linéaire ou ramifié, soit un groupe hydroxy ou les esters correspondants, soit un groupe phénoxy, soit un groupe benzyloxy, soit und pont méthylènedioxy ($-O-CH_2-O-$) lorsque ce substituant est relié avec un carbone voisin, soit un halogène, soit un groupe nitré, soit une fonction amine correspondant à une amine primaire ou secondaire ou tertiaire ou un ammonium quaternaire;
chacun des substituants R9 ou R10 peut représenter soit un atome d'hydrogène, soit un groupe alkyle linéaire ou ramifié, soit un arylalkyle, soit une fonction amine correspondant à une

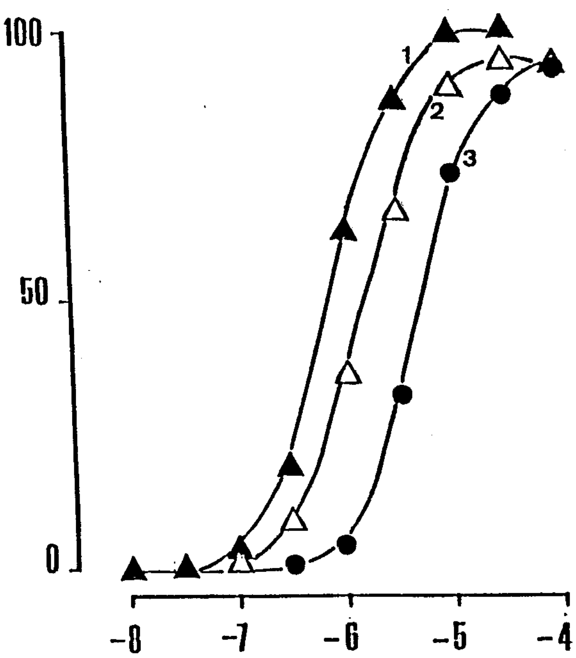


EP 0 236 251 A2

amine primaire ou secondaire ou tertiaire ou un ammonium quaternaire, soit tous les deux un atome d'oxygène;
chacun des substituants R11 ou R12 peut représenter soit un atome d'hydrogène, soit un groupe alkyle linéaire ou ramifié;
R11 et R12 peuvent aussi ensemble former un atome d'oxygène;
R13 peut représenter soit un atome d'hydrogène, soit un groupe alkyle linéaire ou ramifié.

Ils présentent une activité α adrénergique et plus particulièrement une spécificité soit α1 soit α2 adrénergique.

L'invention a égelament pour objet les compositions pharmaceutiques, les médicaments les contenant, ainsi que leurs applications.

UNIVERSITE LOUIS PASTEUR
4, rue Blaise Pascal - 67000 STRASBOURG

DERIVES DES (NITRO-2 OU AMINO-2 BENZYL)-2 TETRAHYDRO-1,2,3,4 ISOQUINOLEINES, AINSI QUE LES PROCEDES D'OBTENTION ET LES COMPOSITIONS PHARMACEUTIQUES LES CONTENANT.

La présente invention concerne les nouveaux dérivés de la (nitro-2 ou amino-2 benzyl)-2 tétrahydro-1,2,3,4 isoquinoléine ainsi que leurs sels, leurs procédés de préparation, leur activité pharmacologique bloquante α2 adrénergique et les compositions pharmaceutiques les renfermant.

Actuellement, de nombreuses molécules α adrénolytiques ont été découvertes. Cependant peu d'entre elles présentent une bonne sélectivité α2 adrénergique. La yohimbine, utilisée couramment comme substance de référence α2 bloquante,par exemple, bien qu'elle ne possède qu'une faible affinité pour les récepteurs α1, présente d'autres effets qui en limitent l'utilisation thérapeutique.

C'est pourquoi, dans la présente invention, on s'est orienté vers la mise au point de molécules α2 bloquantes spécifiques. Les résultats de ces travaux ont permis, par modulation de la benzyl-2. isoquinoléine ( structure chimique différente de celle de la yohimbine), de sélectionner de nouveaux composés d'affinité α2 adrénergiques.

Les substances conformes à l'invention présentent également l'avantage d'être plus hydrosolubles, plus stables et d'un prix de revient moins élevé que la yohimbine.

L'invention a pour objet les dérivés de la (nitro-2 ou amino-2 benzyl)-2

tétrahydro-1,2,3,4 isoquinoléine ainsi que leurs sels d'addition avec les acides minéraux ou organiques, caractérisés en ce qu'ils répondent à la formule générale I :

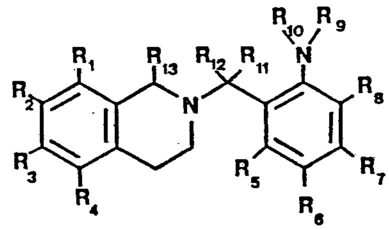

(I)

dans laquelle chacun des substituants R1, R2, R3, R4, R5, R6, R7 ou R8 peut représenter soit un atome d'hydrogène, soit un groupe alkyle linéaire ou ramifié ayant de préférence 1 à 4 atomes de carbone, soit un groupe alcoxy linéaire ou ramifié ayant de préférence 1 à 4 atomes de carbone, soit un groupe hydroxy ou les esters correspondants, soit un groupe phénoxy, soit un groupe benzyloxy, soit un pont méthylènedioxy ($-O-CH_2-O-$) lorsque ce substituant est relié avec un carbone voisin, soit un halogène, soit un groupe nitré, soit une fonction amine correspondant à une amine primaire ou secondaire ou tertiaire ou un ammonium quaternaire;

chacun des substituants R9 ou R10 peut représenter soit un atome d'hydrogène, soit un groupe alkyle linéaire ou ramifié ayant de préférence 1 à 4 atomes de carbone, soit un arylalkyle, soit une fonction amine correspondant à une amine primaire ou secondaire ou tertiaire ou un ammonium quaternaire, soit tous les deux un atome d'oxygène;

chacun des substituants R11 ou R12 peut représenter soit un atome d'hydrogène, soit un groupe alkyle linéaire ou ramifié ayant de préférence 1 à 4 atomes de carbone;

R11 et R12 peuvent aussi ensemble former un atome d'oxygène;

R13 peut représenter soit un atome d'hydrogène, soit un groupe alkyle linéaire ou ramifié ayant de préférence 1 à 4 atomes de carbone;

à l'exclusion du composé où R1 = R4 = R5 = R7 = R8 = R9 = R10 = R11 = R12 = R13 = H, R2 = R3 = $OCH_3$ et R6 = $CH_3$.

Dans la formule générale I et dans ce qui suit, le terme radical alkyle renfermant de préférence 1 à 4 atomes de carbone désigne, par exemple, un radical méthyle et le terme radical alcoxy représente, par exemple, un méthoxy. L'atome d'halogène peut être un atome soit de fluor, soit de chlore, soit de brome, soit d'iode.

Les substituants sur la tétrahydroisoquinoléine peuvent être en toutes positions

sur le noyau benzénique, mais de préférence en position 6 et 7 ou 6 ou 7 , ce qui correspond à R3 et R2.

Les substituants sur le groupe amino-2 ou nitro-2 benzyle peuvent être en toutes positions sur le noyau benzénique, mais, de préférence , en position para et méta ou para ou méta ce qui correspond à R6 et R7.

Les sels d'addition avec les acides minéraux ou organiques peuvent,par exemple, être les sels formés avec l'acide chlorhydrique.

Parmi les produits, objets de l'invention, on peut citer les dérivés répondant à la formule I, ci-dessus, ainsi que leurs sels d'addition avec les sels minéraux ou organiques, caractérisés en ce que, dans ladite formule I, chacun des substituants R9 ou R10 peut représenter soit un atome d'hydrogène, soit un groupe méthyle, soit tous les deux un atome d'oxygène; chacun des substituants R2 ou R3 peut représenter soit un atome d'hydrogène, soit un groupe méthoxy, soit un groupe benzyloxy ; chacun des substituants R6 ou R7 peut représenter soit un atome d'hydrogène, soit un groupe méthoxy , soit un halogène; R2, R3 et/ou R6, R7 peuvent former un pont méthylènedioxy ($-O-CH_2-O-$); R1, R4, R5 et R8 représentent un atome d'hydrogène; R13 peut représenter soit un atome d'hydrogène, soit un méthyle pour donner le produit de formule II :

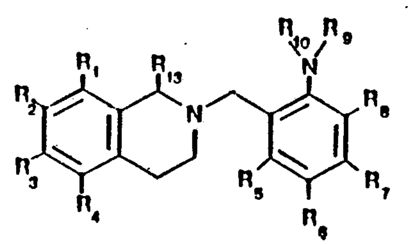

(II)

L'invention a aussi pour objet un procédé de préparation des dérivés, tels que définis par la formule I, ci-dessus, ainsi que leurs sels.

Le composé II peut être obtenu selon les schémas suivants :

<u>1er schéma de synthèse</u> :

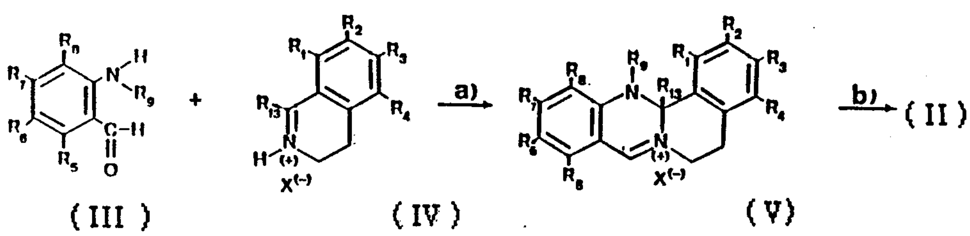


(III) (IV) (V)

chacun des substituants R1, R2, R3, R4, R5, R6, R7 ou R8 peut représenter soit un atome d'hydrogène, soit un groupe alkyle linéaire ou ramifié ayant de préférence 1 à 4 atomes de carbone, soit un groupe alcoxy linéaire ou ramifié ayant de préférence 1 à 4 atomes de carbone, soit un groupe hydroxy ou les esters correspondants, soit un groupe phénoxy, soit un groupe benzyloxy, soit un pont méthylènedioxy (-O-CH2-O-) lorsque ce substituant est relié avec un carbone voisin, soit un halogène, soit un groupe nitré, soit une fonction amine correspondant à une amine primaire ou secondaire ou tertiaire ou ammonium quaternaire;

R9 peut représenter soit un atome d'hydrogène, soit un radical alkyle linéaire ou ramifié ayant de préférence 1 à 4 atomes de carbone, soit un arylalkyle, soit une fonction correspondant à une amine primaire ou secondaire ou tertaire ou un ammonium quaternaire;

R13 peut représenter soit un atome d'hydrogène, soit un groupe alkyle linéaire ou ramifié ayant de préférence 1 à 4 atomes de carbone;

X(-) représente l'anion minéral ou organique en liaison avec l'azote ammonium;

à l'exception du produit où R1 = R4 = R5 = R6 = R7 = R8 = R9 = R13 = H, R2 = R3= OCH3 et X (-) = OH;

Les conditions opératoires a) et b) signifient :

a) Réaction d'un dérivé de l'amino-2 benzaldéhyde (composé III) avec un dérivé d'un sel organique ou minéral de la dihydro-3,4 isoquinoléine (composé IV).

b)Réduction du dérivé de la tétrahydro-5,6,13,13a isoquino[1,2-b] quinazolinium obtenu (composé V) dissous dans le méthanol par le borohydrure de sodium pour obtenir le produit II sous forme de base. A partir de celui- ci, un disel est réalisé en présence d'un acide minéral ou organique.

<u>2ème schéma de synthèse</u> :

Le composé de structure V se transforme en milieu basique organique anhydre en dérivé VI à la condition que R9 et R13 soient un hydrogène :

(V) c)

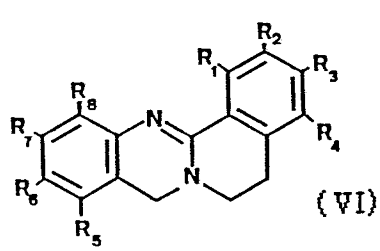

(VI) d) (II)

Chacun des substituants R1, R2, R3, R4, R5, R6, R7 ou R8 peut représenter soit un atome d'hydrogène, soit un groupe alkyle linéaire ou ramifié ayant de préférence 1 à 4 atomes de carbone, soit un groupe alcoxy linéaire ou ramifié ayant de préférence 1 à 4 atomes de carbone, soit un groupe hydroxy ou les esters correspondants, soit un groupe phénoxy, soit un groupe benzyloxy , soit un pont méthylènedioxy (-O-CH2-O-) lorsque ce substituant est relié avec un carbone voisin, soit un halogène, soit un groupe nitré, soit une fonction amine correspondant à une amine primaire ou secondaire ou tertiaire ou un ammonium quaternaire;

Les conditions opératoires c) et d) signifient :

c) le composé V en milieu basique organique anhydre se transforme en composé VI. A partir de celui-ci, le sel est réalisé en présence d'un acide minéral ou organique.

d) Réduction de la base du composé VI obtenu dissous dans le méthanol par le borohydrure de sodium pour obtenir le produit II sous forme de base. A partir de celui-ci, le bisel est réalisé en présence d'un acide minéral ou organique.

La stucture VI a déjà été décrite par William J. Houlihan, Robert E. Manning et Mountain lakes [Sandoz-Wander, Inc., U.S. 3,542,782 (Cl 260-251)] où R1, R4, R5 et R8 sont l'hydrogène; où chacun des substituants R2 ou R3 peut représenter soit l'hydrogène, soit un groupe alkyle, soit un groupe alcoxy; où chacun des substituants R6 ou R7 peut représenter soit l'hydrogène, soit le fluor, soit le chlore, soit un groupe alkyle, soit un groupe alcoxy; les substituants R2, R3 ou/et R6, R7 peuvent former un pont méthylènedioxy (-O-CH2-O-).

Mais la méthode de préparation, conforme à l'invention, est différente de celle publiée dans l'art antérieur.

3 ème schéma de synthèse :

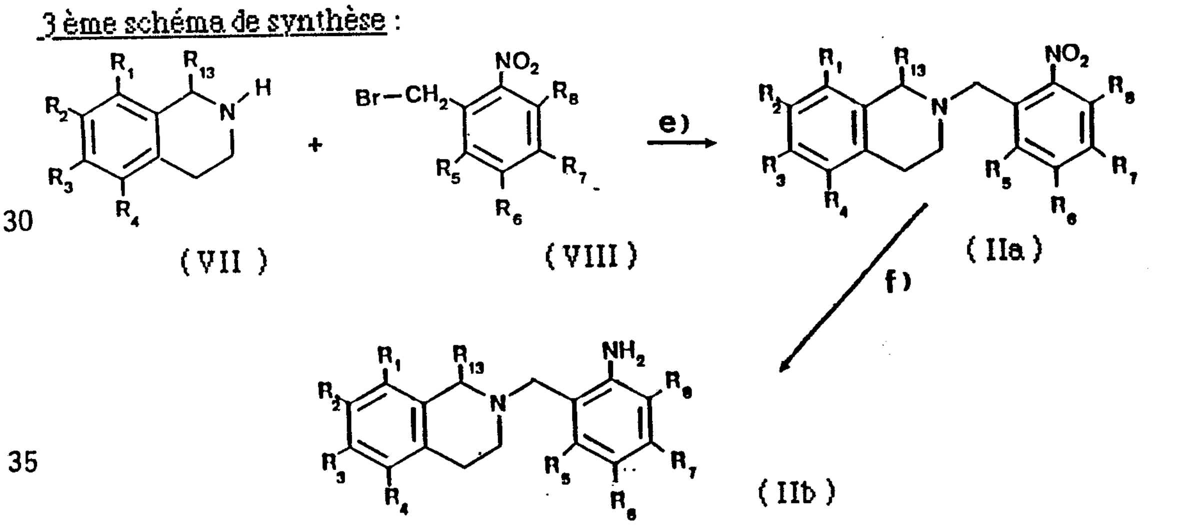


(VII) (VIII) (IIa) (IIb)

Les conditions opératoires e) et f) signifient :

e) Condensation d'un dérivé de la tétrahydro-1,2,3,4 isoquinoléine (composé VII ) avec un dérivé du bromure de nitro-2 benzyle (composé VIII) dans l'éthanol absolu en présence de $CO_3Na_2$ sec. A partir de celui-ci, le sel est réalisé en présence d'un acide minéral ou organique.

f) Réduire la base du composé IIa par le zinc en milieu acide. La base du produit IIb est transformé en disel avec un acide minéral ou organique.

Chacun des substituants R1, R2, R3, R4, R5, R6, R7 ou R8 peut représenter soit un atome d'hydrogène, soit un groupe alkyle linéaire ou ramifié ayant de préférence 1 à 4 atomes de carbone, soit un groupe alcoxy linéaire ou ramifié ayant de préférence 1 à 4 atomes de carbone, soit un groupe hydroxy ou les esters correspondants, soit un groupe phénoxy, soit un groupe benzyloxy, soit un pont méthylènedioxy ($-O-CH_2-O-$) lorsque ce substituant est relié avec un carbone voisin, soit un halogène, soit un groupe nitré, soit une fonction amine correspondant à une amine primaire ou secondaire ou tertiaire ou un ammonium quaternaire;

R13 peut représenter soit un atome d'hydrogène, soit un groupe alkyle linéaire ou ramifié ayant de préférence 1 à 4 atomes de carbone.

à l'exclusion du composé où R1 = R4 = R5 = R7 = R8 = R13 = H et R2 = R3 = $OCH_3$ et R6 = $CH_3$.

Les dérivés, objets de la présente invention, possédent d'intéressantes propriétés pharmacologiques ; ils ont une affinité pour les récepteurs $\alpha 1$ et $\alpha 2$ et sont plus particulièrement sélectifs des récepteurs $\alpha 2$ . L'invention concerne donc une activité $\alpha$ adrénergique.

L'invention va maintenant être décrite plus en détail dans les exemples donnés ci-après à titre non limitatif parmi lesquels:

EXEMPLE 1 : (Amino-2 diméthoxy-4,5 benzyl)-2 méthoxy-6 tétrahydro-1,2,3,4 isoquinoléine
(structure II, R3 = R6 = R7 = $OCH_3$ et R1 = R2 = R4 = R5 = R8 = H
R9 = R10 = R13 = H)

<u>Première étape</u> : Synthèse du chlorure de tétrahydro-5,6,13,13a triméthoxy-3,10,11 isoquino [1,2-b] quinazolinium
(Structure V , R3 = R6 = R7 = $OCH_3$, R1 = R2 = R4 = R5 = R8 = R9 = H
R13 = H)

Dans un ballon muni d'un tube à $CaCl_2$, dissoudre 1 g d'amino-6 vératraldéhyde (0,0055 mole) dans 20 ml d'éthanol absolu. Additionner 1,1 g de chlorhydrate de

méthoxy-6 dihydro-3,4 isoquinoléine (0,0055 mole) dissous dans un minimum d'éthanol absolu. Laisser sous agitation pendant 1 heure. Le produit précipite lentement. Filtrer et laver à l'éthanol absolu.

1,75 g de cristaux oranges sont isolés (Rdt : 87 %)

- PF : 161°C
- La structure de la molécule a été confirmée par des études spectrales :IR (KBr) et RMN (DMSO d6).

<u>Deuxième étape</u> : (amino-2 diméthoxy-4,5 benzyl)-2 méthoxy-6 tétrahydro-1,2,3,4 isoquinoléine (Structure II, R3 = R6 = R7 = $OCH_3$ et R1 = R2 = R4 = R5 = R8 = H R9 = R10 = R13 = H)

Dans un erlenmeyer, dissoudre 1,75 g de chlorure de tétrahydro-5,6,13,13a triméthoxy-3,10,11 isoquino [1,2-b] quinazolinium dans 20 ml de méthanol. Puis additionner par fractions successives 0,20 g de borohydrure de sodium. Laisser en contact sous agitation pendant 30 mn. Puis additionner 10 ml d'eau. Extraire au chloroforme. Sécher la phase chloroformique avec du sulfate de magnésium. Filtrer.

Evaporer le filtrat. Reprendre le résidu par de l'éther éthylique. Le produit cristallise. 0,80 g de cristaux sont isolés (Rdt : 50 %).

- PF = 100°C
- La structure de la molécule a été confirmée par études spectrales : IR ($CHCl_3$) et RMN ($CDCl_3$).

<u>Préparation du dichlorhydrate</u>

Dissoudre 0,80 g de base obtenue dans 5 ml de chloroforme. Additionner HCl gazeux jusqu'à saturation. Laisser sous agitation dans un bain de glace pendant 30 mn. Additionner 1 ml d'éther éthylique anhydre. Le dichlorhydrate précipite. Filtrer. Sécher. Les cristaux fondent à 160°C.

EXEMPLE 2 : ( Amino-2 diméthoxy-4,5 benzyl)-2 diméthoxy-6,7 tétrahydro-1,2,3,4 isoquinoléine ( structure II, R2 = R3 = R6 = R7 =$OCH_3$ , R1 = R4 = R5 = R8 = R9 = H et R10 = R13 = H)

<u>Première étape</u> : Synthèse du chlorure de tétrahydro-5,6,13,13a tétraméthoxy-2,3,10,11 isoquino [1,2-b] quinazolium ( Structure V, R2 = R3 = R6 = R7 = $OCH_3$, R1 = R4 = R5 = R8 = R9 = H et R13 = H )

Elle est réalisée par réaction de l'amino-6 vératraldéhyde sur le chlorhydrate de

la dihydro-3,4 diméthoxy-6,7 isoquinoléine selon les conditions opératoires décrites à la première étape de l'exemple 1.

- Rdt : 86%
- PF : 264°C
- La structure de la molécule a été confirmée par des études spectrales : IR (KBr ) et RMN ( DMSO d6 )

Deuxième étape: ( Amino-2 diméthoxy-4,5 benzyl )-2 diméthoxy-6,7 tétrahydro-1,2,3,4 isoquinoléine
( Structure II, R2 = R3 = R6 = R7 = $OCH_3$, R1 = R4 = R5 = R8 = R9 = H et R10 = R13 = H)

Elle est réalisée de manière analogue à la deuxième étape de l'exemple 1.

- Rdt : 55%
- PF : 154°C
- La structure de la molécule a été confirmée par des études spectales : IR (KBr) et RMN ($CDCl_3$).

Préparation du dichlorhydrate

Il est réalisé de manière analogue à la deuxième étape de l'exemple 1.

EXEMPLE 3 : (Amino-2 benzyl)-2 diméthoxy-6,7 tétrahydro-1,2,3,4 isoquinoléine
(StructureII, R1 = R4 =R5 = R6 = R7 = R8 = R9 = R10 = R13 = H, R2 = R3 = $OCH_3$)

Première étape: Synthèse du chlorure de diméthoxy-2,3 tétrahydro-5,6,13,13a isoquino [1,2-b] quinazolinium
(Structure V, R2 = R3 = $OCH_3$, R1 = R4 = R5 = R6 = R7 = R8 = R9 = H R13 = H )

Dans un ballon muni d'un tube à $CaCl_2$, dissoudre 1 g d'amino-2 benzaldéhyde (0,0083 mole) dans 75 ml d'éthanol absolu. Additionner 1,88 g de chlorhydrate de dihydro-3,4 diméthoxy-6,7 isoquinoléine (0,0083 mole) dissous dans un minimum d'éthanol absolu. Chauffer à ébullition pendant une heure sous agitation. Laisser refroidir . Ajouter 5 ml d'éther éthylique anhydre, le produit précipite. Filtrer . Laver avec de l'alcool absolu.

- Rdt : 55%
- PF = 260°C
- La structure de la molécule a été confirmée par des études spectrales : IR (KBr) et RMN (DMSO d6).

Deuxième étape: (Amino-2 benzyl)-2 diméthoxy-6,7 tétrahydro-1,2,3,4 isoquinoléine

(Structure II, R1 = R4 = R5 = R6 = R7 = R8 = R9 = R10 = R13 = H, R2 = R3 = OCH3)

Elle est réalisée de manière analogue à la deuxième étape de l'exemple 1.

- Rdt : 50%
- PF = 155°C
- La structure de la molécule a été confirmée par des analyses spectrales : IR (KBr ) et RMN ( CDCl3 ).

Préparation du dichlorhydrate

Il est réalisé de manière analogue à la deuxième étape de l'exemple 1.

EXEMPLE 4 :(Diméthoxy-4,5 méthylamino-2 benzyl)-2 diméthoxy-6,7 tétrahydro-1,2,3,4 isoquinoléine
(Structure II , R2 = R3 = R6 = R7 = OCH3, R9 = CH3 et R1 = R4 = R5 = R8 = H R10 = R13 = H)

Première étape : Synthèse du chlorure de méthyl-13 tétraméthoxy-2,3,10,11 tétrahydro-5,6,13,13a isoquino [1,2-b] quinazolinium
(Structure V ,R2 = R3 = R6 = R7 = OCH3, R9 = CH3, R1 = R4 = R5 = H R8 = R 13 = H).

Dans un ballon surmonté d'un réfrigérant muni d'un tube à CaCl2, dissoudre 1 g de diméthoxy-4,5 méthylamino-2 benzaldéhyde (0,0051 mole) dans 20 ml d'éthanol absolu. Puis additionner 1,17 g de chlorhydrate de diméthoxy-6,7 dihydro-3,4 isoquinoléine (0,0051 mole). Chauffer à ébullition pendant 1/2 h sous agitation. Le produit précipite lentement en refroidissant. Filtrer et laver à l'éthanol absolu. 1,20 g de cristaux orange sont isolés (Rdt : 55 %).

- PF = 264 °C
- La structure de la molécule a été confirmée par des analyses spectrales : IR (KBr) et RMN (DMSO d6).

Deuxième étape : (diméthoxy -4,5 méthylamino-2 benzyl)-2 diméthoxy-6,7 tétrahydro-1,2,3,4 isoquinoléine.
(Structure II, R2 = R3 = R6 = R7 = OCH3, R9 = CH3 et R1 = R4 = R5 = R8 = R10 = R13 = H).

Elle est réalisée de manière analogue à la deuxième étape de l'exemple 1.

- Rdt : 55 %
- PF = 172° C (Base)
- La structure de la molécule a été confirmée par études spectrales : IR (KBr) et RMN (CDCl3).

Préparation du dichlorhydrate

Il est réalisé de manière analogue à la deuxième étape de l'exemple 1.

EXEMPLE 5: (Amino-2 diméthoxy-4, 5 benzyl)-2 tétrahydro-1, 2, 3, 4 isoquinoléine
(Structure II, R6 = R7 = OCH3, R1 = R2 = R3 = R4 = R5 = R8 = H R9 = R10 = R13 = H)

Première étape : Synthèse du picrate de diméthoxy-10,11 tétrahydro-5, 6, 13, 13a isoquino [1,2-b] quinazolinium
(Structure V, R6 = R7 = OCH3, R1 = R2 = R3 = R4 = R5 = R8 = R9 = H R13 = H)

Elle est réalisée par réaction de l'amino-6 vératraldéhyde sur le picrate de dihydro-3,4 isoquinoléine selon les conditions opératoires décrites à la première étape de l'exemple 1.

- Rdt : 95 %
- PF : 210°C
- La structure de la molécule a été confirmée par études spectrales IR (KBr) et RMN (CDCl3).

Deuxième étape : Synthèse de la diméthoxy-10,11 dihydro-5,6 8H-isoquino [1,2-b] quinazoline.
(Structure VI, R6 = R7 = OCH3 R1 = R2 = R3 = R4 = R5 = R8 = H)

Dissoudre le produit obtenu lors de la première étape dans un minimum de pyridine anhydre séchée sur KOH. Chauffer à ébullition sous agitation. Puis additionner de la triéthylamine séchée sur KOH. Laisser sous agitation pendant 1h à ébullition. Evaporer sous pression réduite. Reprendre le résidu par de l'eau, alcaliniser par de la soude et extraire au chloroforme. Evaporer la phase chloroformique séchée sur sulfate de magnésium. Reprendre le résidu par un minimum d'éthanol absolu et précipiter le produit avec de l'éther éthylique. Filtrer et laver.

- Rdt : 65 %
- PF : 151°C
- La structure de la molécule a été confirmée par des études spectrales IR (KBr) et RMN (CDCl3).

Préparation du chlorhydrate

Dissoudre la base obtenue dans du chloroforme. Additionner de l'HCl gazeux jusqu'à saturation. Laisser sous agitation dans un bain de glace. Le chlorhydrate précipite. Filtrer. Sécher.

Troisième étape : (Amino-2 diméthoxy-4,5 benzyl)-2 tétrahydro-1, 2, 3, 4 isoquinoléine.

(Structure II, R6 = R7 = OCH3, R1 = R2 = R3 = R4 = R5 = R8 = H R9 = R10 = R13 = H )

Dissoudre dans un erlenmeyer la base du produit obtenu, lors de la deuxième étape précédente, dans du méthanol, puis opérer d'une manière analogue à la deuxième étape de l'exemple 1 ; le dichlorhydrate est réalisé de manière analogue à la deuxième étape de l'exemple 1.

- Rdt : 95 %
- PF = 106°C (base)
- La structure de la molécule a été confirmée par des études spectrales IR (KBr) et RMN (CDCl3).

EXEMPLE 6 : (Chloro-4 nitro-2 benzyl)-2 tétrahydro- 1,2, 3, 4 isoquinoléine
(Structure II, R7 = Cl, R1 = R2 = R3 = R4 = R5 = R6 = R8 = R13 =H, R9 = R10 = O)

Additionner 10 ml d'éthanol absolu à 2,1 g de bromure de chloro-4 nitro-2 benzyle dans un erlenmeyer. Puis ajouter 1,1 g de tétrahydroisoquinoléine base. Laisser sous agitation pendant une heure . Le produit précipite. Filtrer et laver à l'alcool absolu.

Recristalliser dans l'alcool à 95°.

- Rdt : 42 %
- PF = 101° C
- La structure de la molécule a été confirmée par des études spectrales IR (KBr) et RMN (CDCl3).

Préparation du chlorhydrate :

Dissoudre la base obtenue dans du chloroforme. Additionner de l'HCl gazeux jusqu'à saturation. Laisser sous agitation dans un bain de glace. Le chlorhydrate précipite. Filtrer. Sécher.

EXEMPLE 7 : (Amino-2 chloro-4 benzyl)-2 tétrahydro-1, 2, 3, 4 isoquinoléine
(Structure II, R7 = Cl, R1 = R2 = R3 = R4 = R5 = R6 = R8 = H , R9 = R10 = R13 = H)

Dans un ballon muni d'un réfrigérant, dissoudre 1 g de ( chloro-4 nitro-2 benzyl)-2 tétrahydro-1,2,3,4 isoquinoléine dans 10 ml d'acide chlorhydrique à 20 %. Ajouter 1 g de zinc en poudre. Laisser sous agitation à reflux pendant 2 h. Filtrer l'excés de zinc à chaud et laisser refroidir. Le dichlorhydrate du produit aminé précipite.

Filtrer, alcaliniser par de la soude diluée et extraire la base avec du chloroforme. Sécher sur sulfate de magnésium. Evaporer le chloroforme.

- Rdt : 72 %
- PF = 100° C
- La structure de la molécule a été confirmée par des études spectrales IR (KBr) et RMN (CDCl3).

EXEMPLE 8: ( Chloro-4 nitro-2 benzyl)-2 méthoxy-6 tétrahydro-1,2,3,4 isoquinoléine)

( Structure II, R1 = R2 = R4 = R5 = R6 = R8 = R13 = H, R3 = $OCH_3$, R7 = Cl, R9 = R10 = O)

Additionner 10 ml d'éthanol absolu à 2,1g de bromure de chloro-4 nitro-2 benzyle dans un erlenmeyer. Puis ajouter 1,2g de méthoxy-6 tétrahydro-1,2,3,4 isoquinoléine base. Laisser sous agitation pendant une heure. Le produit précipite. Filtrer et laver à l'alcool absolu.

Recristalliser dans l'alcool à 95°.

- Rdt: 45%
- PF = 170°C
- La structure de la molécule a été confirmée par des études spectrales IR (CDCl3) et RMN (CDCl3).

Préparation du chlorhydrate :

Le chlorhydrate est obtenu de la même manière que celle décrite dans l'exemple 6.

EXEMPLE 9: (Amino-2 chloro-4 benzyl)-2 méthoxy-6 tétrahydro-1,2,3,4 isoquinoléine

( Structure II, R7 = Cl, R3 = $OCH_3$, R1 = R2 = R4 = R5 = R6 = R8 = H R9 = R10 = R13 = H )

Dans un ballon muni d'un réfrigérant, dissoudre 1g de ( chloro-4 nitro-2 benzyl)-2 méthoxy-6 tétrahydro-1,2,3,4 isoquinoléine dans 10 ml d'acide acétique à 20%. Ajouter 1g de zinc en poudre. Laisser sous agitation pendant 2 heures. Filtrer l'excès de zinc . Evaporer le maximum de solvant puis extraire au chloroforme après avoir alcaliniser en large excès par de l'ammoniaque diluée. Sécher sur sulfate de magnésium. Evaporer le chloroforme .

Le dichlorhydrate est réalisé de manière analogue à la deuxième étape de l'exemple 1.

- Rdt : 75%
- PF = 160°C ( dichlorhydrate )
- La structure de la molécule a été confirmée par des études spectrales IR (KBr) et RMN ( DMSO d6 ).

Dans le but d'illustrer l'invention, de façon non limitative, des résultats de l'étude pharmacologique qui ont été menée en rapport avec ces substances sont exposées.

Afin de faciliter l'exposé, les produits, objets de l'invention, sont identifiés par le nom de code ISK et les chlorhydrates des substances testées, cités dans ce qui suit, sont identifiés par les noms de code suivants:

ISK4 pour 1'(amino-2 diméthoxy-4,5 benzyl)-2 diméthoxy-6,7 tétrahydro-1,2,3,4 isoquinoléine;

ISK7 pour 1' (amino-2 diméthoxy-4,5 benzyl)-2 méthoxy-6 tétrahydro-1,2,3,4 isoquinoléine;

ISK17 pour 1' (amino-2 benzyl)-2 diméthoxy-6,7 tétrahydro-1,2,3,4 isoquinoléine;

ISK18 pour 1' (amino-2 diméthoxy-4,5 benzyl)-2 tétrahydro-1,2,3,4 isoquinoléine;

ISK23 pour la (diméthoxy-4,5 méthylamino-2 benzyl)-2 diméthoxy-6,7 tétrahydro-1,2,3,4 isoquinoléine;

ISK24 pour la (chloro-4 nitro-2 benzyl)-2 tétrahydro-1,2,3,4 isoquinoléine;

ISK25 pour 1' (amino-2 chloro-4 benzyl)-2 tétrahydro-1,2,3,4 isoquinoléine;

ISK31 pour 1' (amino-2 chloro-4 benzyl)-2 méthoxy-6 tétrahydro-1,2,3,4 isoquinoléine;

Les études pharmacologiques réalisées sur les produits ISK sont décrites ci-dessous et expliquées avec référence aux dessins schématiques annexés, dans lesquels;

la figure 1 représente l'antagonisme de l'ISK7 vis-à-vis du BHT 920 (dichlorhydrate d'allyl-6 amino-2 tétrahydro-5,6,7,8 4H-thiazolo [4,5-d] azépine) sur aorte de rat;

la figure 2 représente l'antagonisme entre l'ISK7,la yohimbine ou la prazosine et la clonidine sur le canal déférent de rat stimulé électriquement; et

la figure 3 représente l'antagonisme entre l'ISK7, la yohimbine ou la prazosine et la phényléphrine sur la concentration du canal déférent de rat.

Dans un premier temps, <u>l'affinité des produits pour les récepteurs α1 et α2 adrénergiques du cortex cérébral de rat est déterminée.</u>

La prazosine [$^3$H], antagoniste spécifique des récepteurs de type α1 et la yohimbine [$^3$H], antagoniste spécifique des récepteurs de type α2 sont utilisées comme ligands radioactifs.

La préparation des homogénats de cortex cérébral se fait à partir de rats mâles

Wistar de 12 semaines. Après décapitation, les cerveaux sont rapidement prélevés et disséqués . Les tissus sont homogénéisés à l'aide d'un ultra-turrax dans un tampon Tris-HCl 50 mM pH 7,5 et centrifugés à 40.000 g pendant 10 minutes. Le culot est repris dans du tampon et recentrifugé de même. L'ensemble des manipulations se fait à une température inférieure à 4° C.

Le culot final est homogénéisé dans le tampon d'incubation maintenu dans la glace fondante (tris-HCl 50 mM pH 7,5) de façon à obtenir une dilution finale du tissu cortical de 1/100 (p/v), ce qui correspond à environ 0,5 mg de protéines par ml.

1 ml de suspension tissulaire est incubé en présence d'une concentration donnée de radioligand (0,2 nM pour la prazosine [$^3$H] et 2 nM pour la yohimbine [$^3$H]) et de diverses concentrations de la molécule étudiée dans un volume final de 2ml. (Les activités spécifiques des radioligands sont de 0,37 -1,11 TBq. mmol$^{-1}$ pour la prazosine [$^3$H], et de 2,59-3,33 TBq. mmol$^{-1}$ pour la yohimbine [$^3$H]).

Les tubes sont incubés à 25°C pendant 30 minutes pour la prazosine [$^3$H], et 20 minutes pour la yohimbine [$^3$H], rapidement filtrés sous vide sur filtres de dénomination commerciale Whatman GF/B et fabriqués par Whatman. Ces filtres sont rincés 4 fois avec 5 ml de tampon d'incubation conservé dans de la glace. La radioactivité retenue sur les filtres est comptée par scintillation liquide.

La fixation non spécifique du ligand radioactif est évaluée en présence de 10 µM de phentolamine.

A partir des courbes d'inhibition de la fixation spécifique d'un ligand à son récepteur par des quantités croissantes d'un produit non radioactif on détermine la constante d'inhibition (Ki) de ce produit. Ces valeurs du Ki sont calculées d'après Cheng et Prussof (Biochem. Pharmacol. 1973, 22, 3099-3108).

Dans le tableau ci-dessous sont consignés les Ki (nM) de quelques composés à titre non limitatif, pour les sites de liaison de la prazosine [$^3$H] et de la yohimbine [$^3$H].

D'autre part, le composé ISK 7 ne présente aucune affinité pour les sites de liaison du dexetimide [$^3$H] (récepteur dopaminergique D2), de la kétansérine [$^3$H] (récepteur sérotonergique), de la pyrilamine [$^3$H] (récepteur histaminique H1) et de la nitrendipine [$^3$H] (bloqueur de l'influx calcique).

Donc, les produits ISK sont revendiqués pour leur activité pharmacologique sélective des récepteurs α adrénergiques et plus particulièrement pour leur spécificité α2 / α1 adrénergique.

| Composés et codes | Prazosine [$^3$H] ($K_D$ = 0,14nM) $K_i$ nM | Yohimbine [$^3$H] ($K_D$ = 10,2nM) $K_i$ nM | $K_i\alpha_1/K_i\alpha_2$ |
|---|---|---|---|
| $ISK_4$ (Structure II : $R_2=R_3=R_6=R_7=OCH_3$) $R_1=R_4=R_5=R_8=H$ $R_9=R_{10}=R_{13}=H$ | 2900 ± 400 | 810 ± 110 | 3,6 |
| $ISK_7$ (Structure II : $R_3=R_6=R_7=OCH_3$) $R_1=R_2=R_4=R_5=R_8=H$ $R_9=R_{10}=R_{13}=H$ | 990 ± 50 | 63 ± 20 | 15,7 |
| $ISK_{17}$ (Structure II : $R_2=R_3=OCH_3$) $R_1=R_4=R_5=R_6=R_7=H$ $R_8=R_9=R_{10}=R_{13}=H$ | 3400 ± 300 | 1700 ± 300 | 2,0 |
| $ISK_{18}$ (Structure II : $R_6=R_7=OCH_3$ $R_1=R_2=R_3=R_4=R_5=H$ $R_8=R_9=R_{10}=R_{13}=H$ | 3220 ± 70 | 220 ± 75 | 14,6 |
| $ISK_{23}$ (Structure II : $R_2=R_3=R_6=R_7=OCH_3$ $R_1=R_4=R_5=R_8=H$ $R_9=R_{13}=H$ $R_{10}=CH_3$ | > 10 000 | 2665 ± 400 | > 3,7 |
| $ISK_{24}$ (Structure II : $R_7=Cl, R_9=R_{10}=O$ $R_1=R_2=R_3=R_4=H$ $R_5=R_6=R_8=R_{13}=H$ | > 30 000 | 2740 ± 1020 | > 11,0 |
| $ISK_{25}$ (Structure II : $R_7=Cl, R_9=R_{10}=H$ $R_1=R_2=R_3=R_4=H$ $R_5=R_6=R_8=R_{13}=H$ | > 30 000 | 1400 ± 375 | > 21,0 |
| $ISK_{31}$ (Structure II : $R_7=Cl, R_9=R_{10}=H$ $R_1=R_2=R_4=R_5=H$ $R_6=R_8=R_{13}=H$ $R_3 = OCH_3$ | 1800 ± 330 | 155 ± 35 | 11,6 |
| Prazosine | 0,15 ± 0.03 | 370 ± 50 | 0,0004 |
| Yohimbine | 350 ± 30 | 17 ± 4 | 20,6 |

Ces résultats représentent la valeur de 3 expériences au moins ± écart type à la moyenne.

Dans un deuxième temps, l'<u>antagonisme des produits ISK vis à vis de l'effet contracturant du BHT 920 sur aorte de rat</u> est démontré.

L'antagonisme de l'ISK 7 vis à vis de l'effet contracturant du BHT 920 (dichlorhydrate d'allyl-6 amino-2 tétrahydro-5,6,7,8 4H-thiazolo [4,5-d] azépine) (agoniste $\alpha$ 2 adrénergique) sur aorte de rat est pris comme exemple.

Il y a antagonisme lorsqu'une substance est capable de s'opposer partiellement ou totalement aux effets d'une substance agoniste.

L'aorte de rats femelles Wistar prélevée rapidement est recueillie dans une solution de Krebs contenant : NaCl 120 mM ; KCl 4,8 mM ; $NaHCO_3$ 25 mM ; glucose 10 mM ; $CaCl_2$ 1,25 mM ; Mg $SO_4$ 1,19 mM ; $KH_2PO_4$ 1,14 mM.

L'aorte débarrassée des tissus conjonctifs et adipeux est découpée en anneaux d'une longueur d' 1 mm environ.

Chaque anneau est installé dans une cuve à organe de 10 ml contenant du liquide de Krebs à 37°, oxygéné par du carbogène (95 % $O_2$ et 5 % $CO_2$) et relié à un capteur isométrique de dénomination commerciale Statham et fabriqué par Gould inc., Medical Product Division, sous une tension de base de 2 g.

L'enregistrement des contractions est réalisé par un polygraphe de dénomination commerciale Beckman et fabriqué par Beckman.

Après une période de repos (1h) entrecoupée de lavages, une première relation effet-concentration est établie en étudiant la contraction provoquée par l'adjonction dans la cuve de concentrations croissantes de BHT 920. Après une nouvelle période de repos entrecoupée de lavages (30 min) où l'organe revient à la tension initiale, l'antagoniste est mis en contact avec le vaisseau pendant 10 minutes ; puis une nouvelle relation effet-concentration avec le BHT 920 est établie. Dans des expériences préliminaires, il a été établi que la première et la deuxième relation effet-concentration de l'agoniste sont identiques en l'absence d'antagoniste. Par contre la présence d'ISK 7 provoque un déplacement sur la droite de la courbe effet-concentration de l'agoniste sans dépression du maximum. Le déplacement augmente avec la concentration d' ISK 7. Ceci démontre que cette substance est antagoniste du BHT 920. La constante de dissociation apparente de l'ISK 7, calculée à partir des expériences illustrée sur la figure 1 pour un antagonisme compétitif suivant Furchgott R.F. (in Catecholamines, ed. by Blaschko et Muscholl, Springer-Verlag, Berlin, 1972 pp. 283-335) est de 1,2 x $10^{-7}$ M pour les deux concentrations utilisées. Cette valeur est comparable à la valeur du Ki obtenue sur les sites de liaison α2 adrénergiques. Ces résultats montrent que l'ISK 7 est un α bloqueur.

La figure 1 représente en abscisse -log(BHT 920) en moles et en ordonnée la contraction en pourcentage de la contraction maximale ; la courbe (1) représente la courbe effet-concentration du BHT en abscence d'ISK 7 et les courbes (2) et (3) en présence de respectivement $10^{-7}$ et $10^{-6}$ moles d'ISK 7.

Par analogie structurale des produits ISK avec l'ISK7, nous revendiquons pour les dérivés, objet de la présente invention, l'activité antagoniste α2 adrénergique.

Dans un troisième temps, <u>l' effet de l' ISK 7 sur les récepteurs α1 et α2 adrénergiques du canal déférent isolé de rat est comparé à des substances de référence, la prazosine et la yohimbine.</u>

L'effet sur les récepteurs de type α1 est mis en évidence par inhibition compétitive de la contraction induite par addition de phényléphrine (agoniste sélectif α1) au liquide de survie, sur les récepteurs de type α2 par un antagonisme compétitif avec l'effet inhibiteur de la clonidine (agoniste sélectif α2) sur la réponse de l'organe à la stimulation dans un champ électrique, suivant la méthode décrite par Starke (Naunyn-Schmiedeberg's Arch. Pharmac.,

1972, 275, 11-23).

Les canaux déférents de rats mâles Wistar de 12 semaines sont prélevés. Chaque canal déférent est placé dans une cuve à organe de 50ml remplie d'une solution de Krebs contenant: NaCl 118mM; KCl 4,7mM; $CaCl_2$ 2,5mM; $KH_2PO_4$ 1,2mM; $MgSO_4$ 1,2mM; $NaHCO_3$ 25mM; glucose 12,5mM.

Le liquide de survie est maintenu à 37°C oxygéné par du carbogène (95% $O_2$ et 5% $CO_2$). Chaque organe est relié à un capteur isométrique de dénomination commerciale Statham et fabriqué par Gould inc., Medical product Division, sous une tension de base de 0,5g. L'enregistrement des contractions est réalisé par un polygraphe de dénomination commerciale Beckman et fabriqué par Beckman.

Pour la caractérisation de l'effet α2 bloqueur, le canal déférent est stimulé électriquement par des impulsions rectangulaires de 2msec de durée, de 30 volts, à la fréquence de 0,2 Hz délivrées par un stimulateur de dénomination commerciale Bioscience (Stimulator 100) et fabriqué par Palmer. Après une période de repos de 20-30 minutes, des concentrations croissantes de clonidine sont ajoutées au milieu de survie toutes les 2 minutes. Cela permet d'établir la relation entre effet et concentration de clonidine. Après la réalisation de deux courbes cumulatives effet-concentration successives séparées par une période de repos de 20 minutes, l'antagoniste est mis en contact avec l'organe isolé pendant 10 minutes et une nouvelle relation effet-concentration est déterminée. Dans des expériences préliminaires, il a été établi que la deuxième et la troisième relation effet-concentration avec l'agoniste sont identiques en l'absence d'antagoniste.

Pour la caractérisation de l'effet α1 bloqueur, l'organe est stimulé par la phényléphrine. La stimulation du canal déférent de rat produit une réponse contractile rapide (le maximum de la contraction est atteint en 30 secondes). Après une période de repos de 30 minutes, des concentrations croissantes de phényléphrine sont ajoutées dans la cuve à organe toutes les 30 secondes. Une première relation effet-concentration est établie. Après un lavage et une nouvelle période de repos de 15 minutes où l'organe revient à la tension initiale, une deuxième relation effet-concentration est effectuée. L'antagoniste est mis au contact du tissu revenu à sa tension de base après lavage et une troisième relation effet concentration est déterminée. Dans des expériences préliminaires il a été établi que la deuxième et la troisième relation effet-concentration de l'agoniste sont identiques en l'absence d'antagoniste.

L'évaluation quantitative de l'antagonisme est effectuée par le calcul du $pA_2$

selon Arunlakshana et Schild (Br. J. Pharmacol. Chemother., 1959,14, 48-58).

La constante de dissociation apparente (KD), calculée selon Furchgott, est le rapport entre la concentration molaire d'antagoniste et le rapport de doses equiactives d'agoniste (en présence et en l'absence d'antagoniste) -1. Cette constante est calculée lorsque la détermination du pA2 n'est pas possible (quand la pente de la relation de Schild est différente de 1).

La figure 2 montre que l'effet inhibiteur de la clonidine sur la réponse contractile du canal déférent stimulé est antagonisé par l'ISK 7 et les substances de référence (yohimbine et prazosine). L'ISK 7, la yohimbine et la prazosine produisent un déplacement vers la droite des courbes effet-concentration de l'agoniste sans diminution de l'effet maximal de ce dernier. Les trois schémas de la figure 2 représentent l'antagonisme de l'ISK 7 ( fig.2.A, aux concentrations de 0; 100; 300; 1000; 3000nM),de la prazosine ( fig.2.B, aux concentrations 0; 1000; 3000; 10000nM) ou de la yohimbine ( fig.2.C, aux concentrations 0; 30; 100; 300; 1000nM) en fonction du -log (clonidine) en moles sur le canal déférent de rat stimulé électriquement. L'effet (inhibition de la réponse contractile) est exprimé en pourcentage de l'effet maximal .Ce déplacement augmente avec la concentration des antagonistes.

Les valeurs de pA2 pour les récepteurs α-adrénergiques présynaptiques de l'ISK 7 et de la yohimbine sont calculées et consignées dans le tableau ci-dessous.

| substances | antagonisme avec la clonidine sur la réponse à la stimulation électrique | | antagonisme avec la phénylephrine | |
|---|---|---|---|---|
| | $pA_2$ | pente | $pA_2$ | pente |
| ISK7 | 6.7 | 1.05 | 5.54 (a) | |
| Yohimbine | 7.59 | 1.15 | 6.08 | 0.85 |
| Prazosine | 5.14 (a) | | 8.34 | 0.99 |

(a) -log de $K_B$ le $pA_2$ ne pouvant être calculé en raison de la pente << 1

Pour ces deux substances, la relation de Schild est linéaire avec une pente non significativement différente de 1. Dans le cas de la prazosine, la pente de la relation de Schild est très différente de 1, la constante de dissociation apparente KD est calculée dans ce cas.

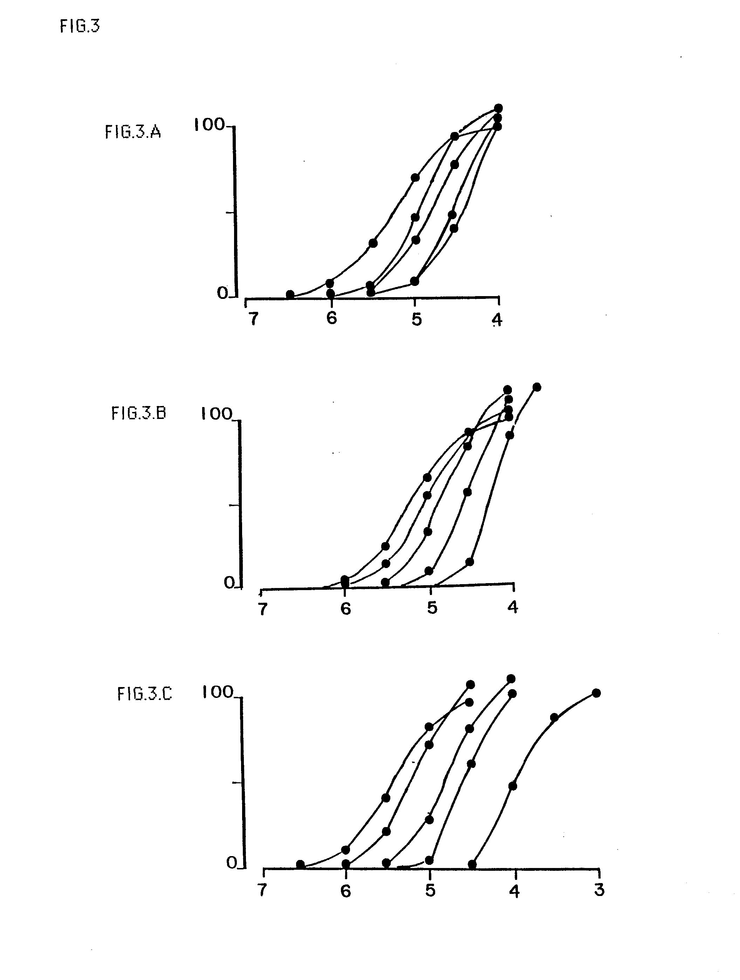

La figure 3 montre qu'en présence d'ISK 7, de yohimbine ou de prazosine, les courbes effet-concentration de phényléphrine sont déplacées vers la droite

sans dépression du maximum . Les trois schémas de la figure 3 représentent en abscisse -log(phényléphrine) en moles et en ordonnée la contraction en pourcentage de la contraction maximale en présence respectivement de ISK 7 (fig.3.A, aux concentrations de 0; 1; 3; 10 et 30µM ), de la yohimbine (fig.3.B, aux concentrations de 0; 0,3; 1; 3; 10µM) , de la prazosine (fig.3.C, aux concentrations de 0; 3; 10; 30; 100nM). Le déplacement augmente avec les concentrations d'antagoniste. Les valeurs de $pA_2$ sont calculées et consignées dans le tableau ci-dessus.

La relation de Schild est linéaire avec une pente de 0,99 pour la prazosine et de 0,85 pour la yohimbine. Dans le cas de l'ISK 7 la pente de la relation de Schild est très différente de 1; c'est la constante de dissociation apparente $K_D$ qui est calculée.

Les résultats exposés ci-dessus montrent que l'ISK 7 est un antagoniste de type α2 adrénergique sélectif sur le canal déférent isolé de rat . Ces résultats sont en accord avec ceux obtenus sur l'aorte isolée de rat puisque le $pA_2$ de l'ISK 7 est respectivement de 6,7 sur le canal déférent et de 6,9 sur l'aorte. Ils confirment, en outre, à l'aide d'une méthode pharmacologique qui permet la caractérisation fonctionnelle des récepteurs, les conclusions de l'étude biochimique de la compétition entre l'ISK 7 et les ligands α-adrénergiques, réalisée à l'aide des techniques de liaison spécifique.

En effet, la présente invention montre que l'ISK 7 n'inhibe l'effet de l'agoniste α1 adrénergique, la phényléphrine, qu'à concentrations très sensiblement plus élevées que les concentrations auxquelles il inhibe compétitivement l'effet de l'agoniste α2 sélectif, la clonidine. L'action α2 bloquante de l'ISK 7 ainsi mise en évidence est habituellement interprétée comme une action pré-jonctionnelle se traduisant par la levée de l'inhibition exercée par l'activation des récepteurs α2 sur la libération de noradrénaline provoquée par la stimulation électrique des terminaisons sympathiques.

Les applications thérapeutiques de l'invention sont celles qui découlent du blocage des récepteurs α2 adrénergiques. Par exemple, on sait qu'il existe des récepteurs de cette nature au niveau du système nerveux central, au niveau des muscles lisses vasculaires, au niveau des plaquettes sanguines, au niveau du pancréas endocrine et au niveau du procès ciliaire.

Le blocage de ces récepteurs α2 adrénergiques peut entraîner des modifications des transmissions catécholaminergiques au niveau du système nerveux central,

une diminution de l'effet vasoconstricteur des catécholamines au niveau des muscles lisses vasculaires, une inhibition de l'agrégation des plaquettes sanguines, une régulation de la sécrétion d'insuline par le pancréas endocrine et de ce fait un effet hypoglycémiant, une diminution de la pression intra-oculaire au niveau du procès ciliaire.

Les indications thérapeutiques correspondantes peuvent être notamment la dépression mentale, certaines hypertensions artérielles, certains troubles de l'hémostase, certains diabètes et le traitement du glaucome.

La présente invention a, par conséquent, également pour objet, les médicaments, caractérisés en ce qu'ils contiennent les nouveaux dérivés de l'(amino-2 ou nitro-2 benzyl)-2 tétrahydro-1,2,3,4 isoquinoléine de formule I, comme principes actifs. En particulier, ces médicaments peuvent être préférentiellement utilisés dans les applications suivantes:

dans la dépression mentale, les principes actifs agissant par modifications des transmissions catécholaminergiques;

dans certaines hypertensions artérielles, les principes actifs agissant par inhibition de l'effet vasoconstricteur des catécholamines;

dans certains troubles de l'hémostase, les principes actifs inhibant l'agrégation des plaquettes sanguines;

dans certains diabètes, les principes actifs régulant la sécrétion d'insuline et la glycémie;

dans le glaucome, les principes actifs diminuant la pression intra-oculaire.

Bien entendu, la présente invention ne se limite nullement aux indications et aux applications décrites. D'autres indications et applications sont possibles, ainsi que des modifications aux indications et aux applications décrites, sans sortir pour autant du domaine de l'invention.

**REVENDICATIONS**

1. Nouveaux dérivés de l'(amino-2 ou nitro-2 benzyl)-2 tétrahydro-1,2,3,4 isoquinoléine, ainsi que leurs sels d'addition avec des acides minéraux ou organiques, caractérisés en ce qu'ils répondent à la formule générale I :

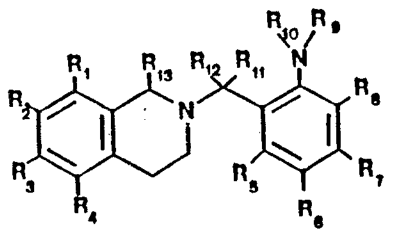

(I)

dans laquelle chacun des substituants R1, R2, R3, R4, R5, R6, R7 ou R8 peut représenter soit un atome d'hydrogène, soit un groupe alkyle linéaire ou ramifié ayant de préférence 1 à 4 atomes de carbone, soit un groupe alcoxy linéaire ou ramifié ayant de préférence 1 à 4 atomes de carbone, soit un groupe hydroxy ou les esters correspondants, soit un groupe phénoxy, soit un groupe benzyloxy , soit un pont méthylènedioxy ($-O-CH_2-O-$) lorsque ce substituant est relié avec un carbone voisin, soit un halogène, soit un groupe nitré, soit une fonction amine correspondant à une amine primaire ou secondaire ou tertiaire ou un ammonium quaternaire;
chacun des substituants R9 ou R10 peut représenter soit un atome d'hydrogène, soit un groupe alkyle linéaire ou ramifié ayant de préférence 1 à 4 atomes de carbone, soit un arylalkyle, soit une fonction amine correspondant à une amine primaire ou secondaire ou tertiaire ou un ammonium quaternaire, soit tous les deux un atome d'oxygène;
chacun des substituants R11 ou R12 peut représenter soit un atome

d'hydrogène, soit un groupe alkyle linéaire ou ramifié ayant de préférence 1 à 4 atomes de carbone;

R11 et R12 peuvent aussi ensemble former un atome d'oxygène;

R13 peut représenter soit un atome d'hydrogène, soit un groupe alkyle linéaire ou ramifié ayant de préférence 1 à 4 atomes de carbone;

à l'exclusion du composé où R1 = R4 = R5 = R7 = R8 = R9 = R10 = R11 = R12 = R13 = H, R2 = R3 = $OCH_3$ et R6 = $CH_3$.

2. Nouveaux dérivés, en tant qu'intermédiaires de synthèse, N-substitués ou non du sel de tétrahydro- 5, 6, 13, 13a isoquino [1,2-b] quinazolinium, caractérisés en ce qu'ils répondent à la formule générale V :

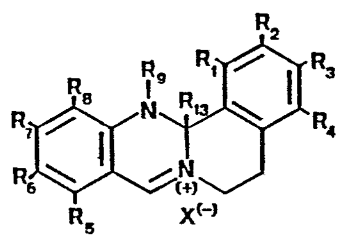

(V)

dans laquelle chacun des substituants R1, R2, R3, R4, R5, R6, R7, ou R8 peut représenter soit un atome d'hydrogène, soit un groupe alkyle linéaire ou ramifié ayant de préférence 1 à 4 atomes de carbone, soit un groupe alcoxy linéaire ou ramifié ayant de préférence 1 à 4 atomes de carbone, soit un groupe hydroxy ou les esters correspondants, soit un groupe phénoxy, soit un groupe benzyloxy, soit un pont méthylènedioxy ($-O-CH_2-O-$) lorsque ce substituant est relié avec un carbone voisin, soit un halogène, soit un groupe nitré, soit une fonction amine correspondant à une amine primaire ou secondaire ou tertiaire ou ammonium quaternaire;

R9 peut représenter soit un atome d'hydrogène, soit un radical alkyle linéaire ou ramifié ayant de préférence 1 à 4 atomes de carbone, soit un arylalkyle ou une fonction correspondant à une amine primaire ou secondaire ou tertaire ou un ammonium quaternaire;

R13 peut représenter soit un atome d'hydrogène, soit un groupe alkyle linéaire ou ramifié ayant de préférence 1 à 4 atomes de carbone;

$X^{(-)}$ représente l'anion minéral ou organique en liaison avec l'azote ammonium;

à l'exception du produit où R2 = R3 = $OCH_3$ et R1= R4= R5= R6= R7= R8= R9 = R13 = H, $X^{(-)}$ = OH;

3. Dérivés de la dihydro-5,6 8H-isoquino [1,2-b] quinazoline, ainsi que

leurs sels d'addition avec les acides minéraux ou organiques, caractérisés en ce qu'ils répondent à la formule générale VI, en tant qu'intermédiaires de synthèse

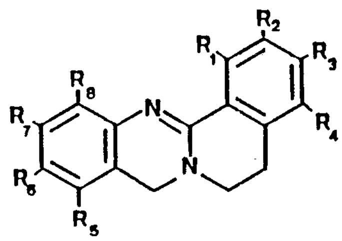

(VI)

dans laquelle chacun des substituants R1, R2, R3, R4, R5, R6, R7 ou R8 peut représenter soit un atome d'hydrogène, soit un groupe alkyle linéaire ou ramifié ayant de préférence 1 à 4 atomes de carbone, soit un groupe alcoxy linéaire ou ramifié ayant de préférence 1 à 4 atomes de carbone, soit un groupe hydroxy ou les esters correspondants, soit un groupe phénoxy, soit un groupe benzyloxy, soit un pont méthylènedioxy ($-O-CH_2-O-$) lorsque ce substituant est relié avec un carbone voisin, soit un halogène, soit un groupe nitré, soit une fonction amine correspondant à une amine primaire ou secondaire ou tertiaire ou un ammonium quaternaire;

à l'exclusion des composés où R1, R4, R5 et R8 sont l'hydrogène;
chacun des substituants R2 ou R3 peut représenter soit l'hydrogène, soit un groupe alkyle, soit un groupe alcoxy ;
chacun des substituants R6 ou R7 peut représenter soit l'hydrogène, soit le fluor, soit le chlore, soit un groupe alkyle, soit un groupe alcoxy ;
R2, R3 et/ou R6, R7 peuvent former un pont méthylènedioxy.

4. Procédé de préparation des dérivés de formule V, selon la revendication 2, obtenus par réaction d'un dérivé de l'amino-2 benzaldéhyde sur un dérivé d'un sel organique ou minéral de la dihydro- 3,4 isoquinoléine.

5. Procédé de préparation des dérivés de formule VI, selon la revendication 3, obtenus à partir du produit de formule V, selon la revendication 2, qui se transforme en milieu basique organique anhydre en dérivé VI ainsi que leurs sels d'addition avec les acides minéraux ou organiques.

6. Procédé de préparation des dérivés de formule I, selon la revendication 1, obtenus par réduction des produits de formule V, selon la revendication 4,

avec le borohydrure de sodium dans le méthanol ainsi que leurs sels d'addition avec les acides minéraux ou organiques.

7. Procédé de préparation des dérivés de formule I, selon la revendication 1, obtenus par réduction des produits de formule VI, selon la revendication 5, avec le borohydrure de sodium dans le méthanol, ainsi que leurs sels d'addition avec les acides minéraux ou organiques.

8. Procédé de préparation des dérivés de formule I ainsi que leurs sels d'addition avec les acides minéraux ou organiques , selon la revendication 1, obtenus par condensation d'un dérivé de la tétrahydro-1,2,3,4 isoquinoléine avec un dérivé du bromure de nitro-2 benzyle dans 1' éthanol absolu en présence de $CO_3Na_2$ sec suivi éventuellement d'une réduction par le zinc en milieu acide.

9. Composé, selon l'une quelconque des revendications 1, 6 à 8, de formule I, caractérisé en ce qu'il représente avantageusement la structure suivante: l'(amino - 2 diméthoxy- 4,5 benzyl)-2 méthoxy-6 tétrahydro-1,2,3,4 isoquinoléine, ainsi que ces sels d'acides d'addition avec les acides minéraux ou organiques.

10. Composés, selon l'une quelconque des revendications 1,6 à 8 de formule I, caractérisés en ce qu'ils représentent l'une des structures suivantes :

(amino-2 diméthoxy-4,5 benzyl)-2 diméthoxy-6,7 tétrahydro-1,2,3,4 isoquinoléine;

(amino-2 benzyl)-2 diméthoxy -6,7 tétrahydro - 1,2,3,4 isoquinoléine;

(amino-2 diméthoxy-4,5 benzyl)-2 tétrahydro-1,2,3,4 isoquinoléine;

(diméthoxy-4,5méthylamino-2benzyl)-2diméthoxy-6,7tétrahydro-1,2,3,4 isoquinoléine;

(chloro-4 nitro-2 benzyl)-2 tétrahydro-1,2,3,4 isoquinoléine;

( amino-2 chloro-4 benzyl)-2 tétrahydro-1,2,3,4 isoquinoléine;

( chloro-4 nitro-2 benzyl )-2 méthoxy-6 tétrahydro-1,2,3,4 isoquinoléine;

( amino-2 chloro-4 benzyl)-2 méthoxy-6 tétrahydro-1,2,3,4 isoquinoléine;

ainsi que leurs sels d'addition avec les acides minéraux ou organiques.

11. Composés de formule I, selon l'une quelconque des revendications 1, 6 à 10, ainsi que leurs sels d'addition avec des acides minéraux ou organiques pour leur activité pharmacologique sélective α adrénergique et plus particulièrement pour leur spécificité soit α1 soit α2 adrénergique.

12. Composé, de formule I, selon la revendication 9, caractérisé pour son

activité pharmacologique liée à sa spécificité α2/α1 adrénergique et sa sélectivité proche de celle de la yohimbine.

13. Composés, selon l'une quelconque des revendications 1, 9 et 10, ainsi que leurs sels d'addition avec des acides minéraux ou organiques pour leur activité antagoniste α2 adrénergique.

14. Médicaments utilisés dans la dépression mentale, caractérisés en ce qu'ils contiennent les nouveaux dérivés de l'(amino-2 ou nitro-2 benzyl)-2 tétrahydro-1,2,3,4 isoquinoléine de formule I, comme principes actifs, tels que définis dans l'une quelconque des revendications 1,9 à 13, ou par leurs sels d'addition avec les acides pharmaceutiquement acceptables, lesdits nouveaux dérivés agissant par modifications des transmissions catécholaminergiques.

15. Médicaments utilisés dans certaines hypertensions artérielles, caractérisés en ce qu'ils contiennent les nouveaux dérivés de l'(amino-2 ou nitro-2 benzyl)-2 tétrahydro-1,2,3,4 isoquinoléine de formule I, comme principes actifs, tels que définis dans l'une quelconque des revendications 1,9 à 13, ou par leurs sels d'addition avec les acides pharmaceutiquement acceptables, lesdits nouveaux dérivés agissant par diminution de l'effet vasoconstricteur des catécholamines.

16. Médicaments utilisés dans certains troubles de l'hémostase, caractérisés en ce qu'ils contiennent les nouveaux dérivés de l'(amino-2 ou nitro-2 benzyl)-2 tétrahydro-1,2,3,4 isoquinoléine de formule I, comme principes actifs, tels que définis dans l'une quelconque des revendications 1, 9 à 13, ou par leurs sels d'addition avec les acides pharmaceutiquement acceptables, lesdits nouveaux dérivés inhibant l'agrégation des plaquettes sanguines.

17. Médicaments utilisés dans certains diabètes, caractérisés en ce qu'ils contiennent les nouveaux dérivés de l'(amino-2 ou nitro-2 benzyl)-2 tétrahydro-1,2,3,4 isoquinoléine de formule I, comme principes actifs, tels que définis dans l'une quelconque des revendications 1, 9 à 13, ou par leurs sels d'addition avec les acides pharmaceutiquement acceptables, lesdits nouveaux dérivés régulant la sécrétion d'insuline et la glycémie.

18. Médicaments utilisés dans le glaucome, caractérisés en ce qu'ils contiennent les nouveaux dérivés de l'(amino-2 ou nitro-2 benzyl)-2 tétrahydro-1,2,3,4 isoquinoléine de formule I, comme principes actifs, tels que définis dans l'une quelconque des revendications 1, 9 à 13, ou par leurs sels d'addition avec les acides pharmaceutiquement acceptables, lesdits nouveaux dérivés diminuant la pression intra-oculaire.

FIG.1

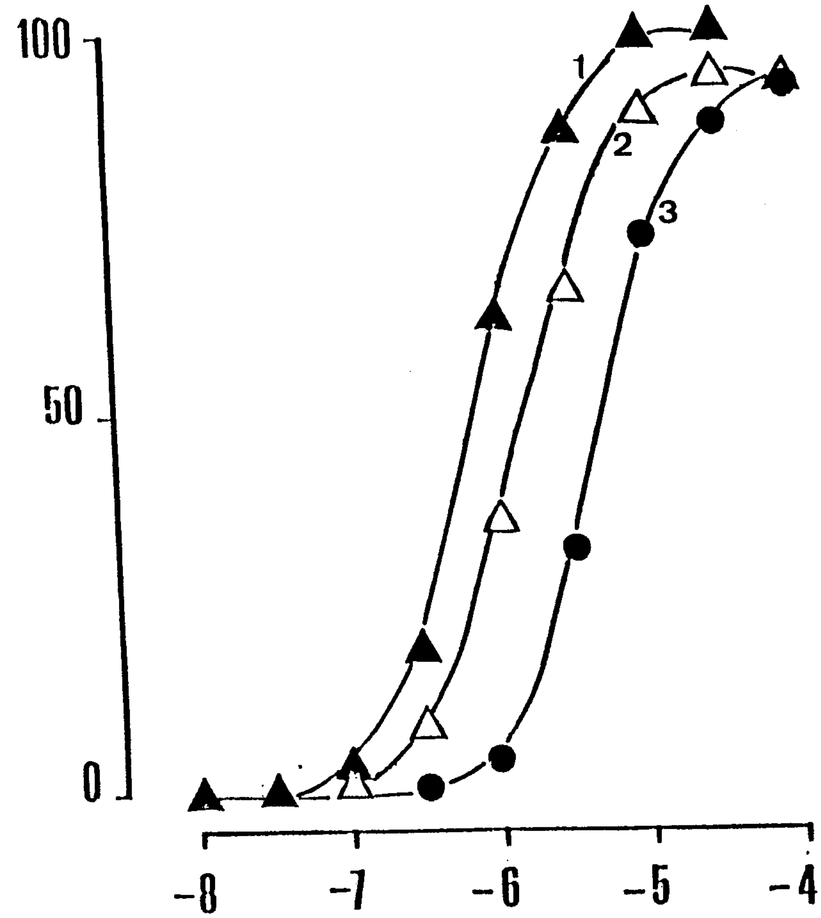

FIG.2

FIG.2.A

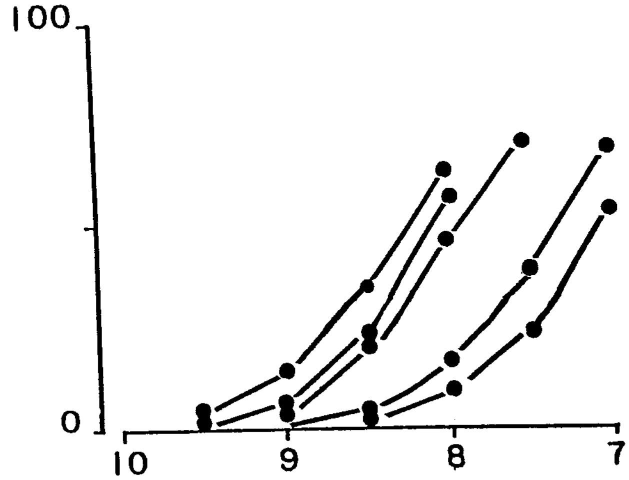


FIG.2.B

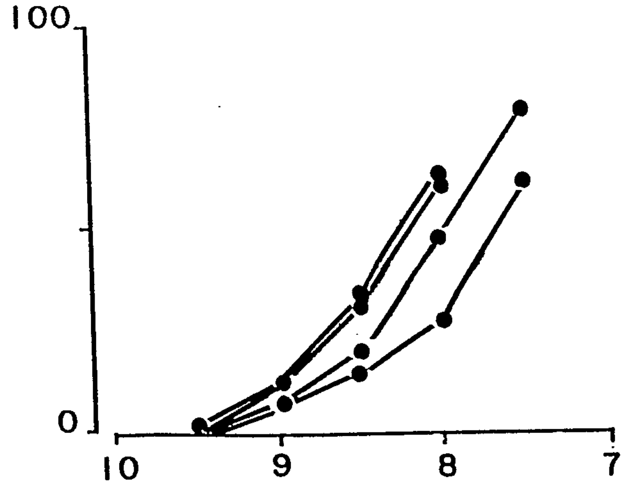


FIG.2.C

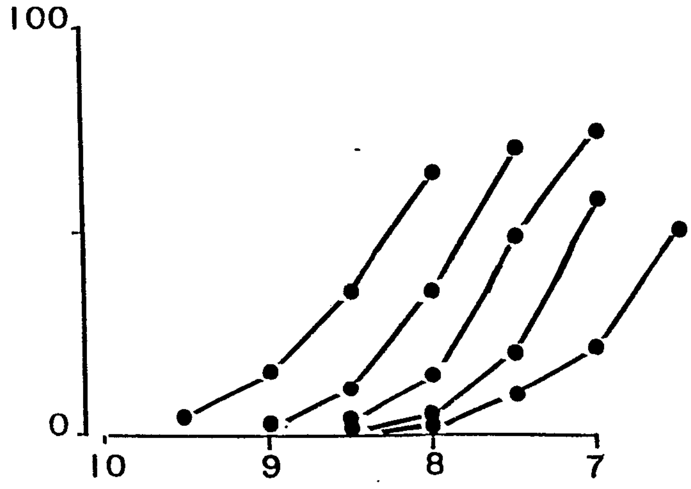

FIG.3

FIG.3.A

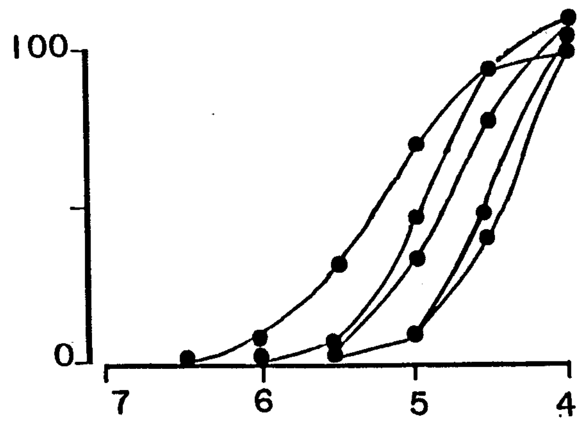


FIG.3.B

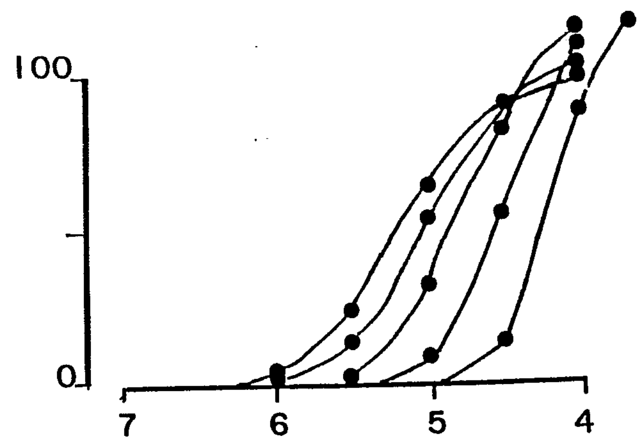


FIG.3.C